**Europäisches Patentamt**

**European Patent Office**

**Office européen des brevets**

(11) Veröffentlichungsnummer: **0 396 976**
**A2**

(12) **EUROPÄISCHE PATENTANMELDUNG**

(21) Anmeldenummer: 90108012.7

(22) Anmeldetag: 27.04.90

(51) Int. Cl.⁵: **C07C 265/14, C07C 263/04,**
**C07C 263/20**

(30) Priorität: 10.05.89 DE 3915183

(43) Veröffentlichungstag der Anmeldung:
14.11.90 Patentblatt 90/46

(84) Benannte Vertragsstaaten:
**BE DE ES FR GB IT NL**

(71) Anmelder: **BAYER AG**

**D-5090 Leverkusen 1 Bayerwerk(DE)**

(72) Erfinder: **Friederichs, Wolfgang, Dr.**
**Geilenkircher Strasse 45**
**D-5000 Köln 41(DE)**
Erfinder: **Hammen, Günter, Dr.**
**Bunzlauer Weg 13**
**D-4049 Rommerskirchen 1(DE)**
Erfinder: **Knöfel, Hartmut, Dr.**
**Dülmener Weg 21**
**D-5068 Odenthal-Erberich(DE)**

(54) Verfahren zur Herstellung von Polyisocyanaten.

(57) Die Erfindung betrifft ein neues Verfahren zur Herstellung von organischen Polyisocyanaten durch thermische Spaltung der ihnen zugrundeliegenden Carbaminsäureester.

EP 0 396 976 A2

## Verfahren zur Herstellung von Polyisocyanaten

Die Erfindung betrifft ein neues Verfahren zur Herstellung von organischen Polyisocyanaten durch thermische Spaltung der ihnen zugrundeliegenden Carbaminsäureester.

Es ist seit langem bekannt, daß N-substituierte Urethane thermisch in der Gasphase oder in flüssiger Phase in Isocyanate und Alkohol spaltbar sind (A.W. Hofmann, Ber. Dtsch. Chem. Ges. 3, 653 (1870); H. Schiff, Ber. Dtsch. Chem. Ges. 3, 649 (1870)).

In der US-PS 24 09 712 ist ein Verfahren beschrieben, bei dem die bei der Spaltung von Carbaminsäureestern in Substanz entstehenden Produkte durch Einleiten in ein Cyclohexan/Wasser-Gemisch an der Rekombination gehindert werden sollen. Dieses Verfahren liefert infolge partieller Hydrolyse der entstehenden Isocyanate an der Phasengrenze nur mäßige Isocyanatausbeuten.

In Anwesenheit inerter hochsiedender Lösungsmittel verlaufen die Verfahren z.B. gemäß US-PS 39 62 302 und US-PS 39 19 278. Dabei werden die beiden Spaltprodukte, also Alkohol und Isocyanat, gemeinsam kontinuierlich aus dem Spaltmedium abdestilliert und durch fraktionierte Kondensation getrennt. Nachteilig bei diesem Verfahren sind der hohe technische Aufwand zur Trennung von Alkohol- und Isocyanatdampf und die nur mäßige Ausbeute. Leichtflüchtige Isocyanate lassen sich infolge der starken Verdünnung und des daraus resultierenden geringen Partialdampfdrucks nur schwer aus dem Spaltmedium abdestillieren. Schwerflüchtige Isocyanate, z.B. Polyisocyanate der Diphenylmethanreihe lassen sich nach diesem Verfahren nicht herstellen.

Bei dem Verfahren gemäß US-PS 39 19 279, DE-OS 26 35 490 oder DE-OS 29 42 543 werden zur Erhöhung der Raum/Zeit-Ausbeute homogene bzw. heterogene Katalysatoren mitverwendet. Gemäß EP-A-00 61 013 sollen durch Zusatz von stabilisierenden Zusätzen Isocyanatfolgereaktionen unterdrückt werden. Die Schwierigkeiten, die sich bei der erforderlichen Destillation der Isocyanate ergeben, lassen sich jedoch durch solche Zusätze nicht verringern.

Es war die der Erfindung zugrundeliegende Aufgabe, ein neues Verfahren zur Herstellung von organischen Polyisocyanaten durch thermische Spaltung der den Polyisocyanaten entsprechenden Carbaminsäureester zur Verfügung zu stellen, bei welchem die entstehenden Polyisocyanate unter Verhinderung einer Rekombination mit dem gebildeten Alkohol schonend aus dem Spaltmedium isoliert werden, und welches insbesondere auch zur Herstellung von schwerflüchtigen Polyisocyanaten geeignet ist.

Diese Aufgabe konnte mit dem nachfolgend näher erläuterten erfindungsgemäßen Verfahren gelöst werden.

Gegenstand der Erfindung ist ein Verfahren zur Herstellung von Polyisocyanaten durch thermische Spaltung der den Polyisocyanaten entsprechenden N-substituierten Carbaminsäureester in Lösung in einem als Spaltmedium dienenden Lösungsmittel oder Lösungsmittelgemisch bei oberhalb 150° C liegenden Temperaturen und unter fortlaufender destillativer Entfernung des bei der Spaltung entstehenden Alkohols, dadurch gekennzeichnet, daß man

A) den Carbaminsäureester in einem Lösungsmittel oder Lösungsmittelgemisch löst, das

a) die Carbaminsäureester zu lösen vermag,

b) bei der Spaltungstemperatur stabil und gegenüber den Carbaminsäureestern und den gebildeten Isocyanaten inert ist, und

c) mit dem gemäß B) eingesetzten Extraktionsmittel mindestens eine Mischungslücke aufweist, und

B) das im Spaltmedium verbleibende, gegebenenfalls durch fraktionierte Destillation im Sumpf angereicherte Polyisocyanat durch Extraktion mit einem Extraktionsmittel, welches mit dem Spaltmedium nicht unbegrenzt mischbar ist und für das Polyisocyanat ein Lösungsmittel darstellt, aus dem Spaltmedium entfernt und gegebenenfalls anschließend die dabei anfallende Lösung des Polyisocyanats in dem Extraktionsmittel destillativ aufarbeitet, und

C) den nicht extrahierten Teil des Reaktionsgemisches zurückführt, vorzugsweise in die Spaltungsreaktion.

Bei den beim erfindungsgemäßen Verfahren einzusetzenden Carbaminsäureestern handelt es sich um Verbindungen oder Gemische von Verbindungen der Formel

$$R^1(NHCOOR^2)_n$$

in welcher

$R^1$ für einen gegebenenfalls inerte Substituenten aufweisenden aliphatischen Kohlenwasserstoffrest mit insgesamt 4 bis 18 Kohlenstoffatomen, einen gegebenenfalls inerte Substituenten aufweisenden cycloaliphatischen Kohlenwasserstoffrest mit insgesamt 6 bis 25 Kohlenstoffatomen, einen gegebenenfalls inerte Substituenten aufweisenden araliphatischen Kohlenwasserstoffrest mit insgesamt 7 bis 25 Kohlenstoffatomen oder einen gegebenenfalls inerte Substituenten aufweisenden aromatischen Kohlenwasserstoffrest mit insgesamt 6 bis 30 Kohlenstoffatomen steht,

$R^2$ für einen Alkylrest mit 1 bis 18 Kohlenstoffatomen, Cycloalkylrest mit 5 bis 15 Kohlenstoffatomen, Aralkylrest mit 7 bis 10 Kohlenstoffatomen oder Arylrest mit 6 bis 10 Kohlenstoffatomen steht, und

n für eine ganze Zahl von 2 bis 5 steht,

mit der Maßgabe, daß die dem Rest $R^2$ entsprechenden Alkohole $R^2$-OH bei Normaldruck einen mindestens 10°C unterhalb des Siedepunkts des als Spaltmedium verwendeten Lösungsmittels und des dem Rest $R^1$ entsprechenden Polyisocyanat $R^1$(NCO)$_n$ liegenden Siedepunkt aufweisen.

Vorzugsweise werden beim erfindungsgemäßen Verfahren solche Carbaminsäureester der genannten Formel eingesetzt, für welche

$R^1$ für einen aliphatischen Kohlenwasserstoffrest mit 4 bis 12, insbesondere 6 bis 10 Kohlenstoffatomen, einen cycloaliphatischen Kohlenwasserstoffrest mit 6 bis 15 Kohlenstoffatomen, einen Xylylenrest oder einen aromatischen, gegebenenfalls Methylsubstituenten und/oder Methylenbrücken aufweisenden Kohlenwasserstoffrest mit insgesamt 7 bis 30 Kohlenstoffatomen steht,

$R^2$ für einen Alkylrest mit 1 bis 6, insbesondere 1 bis 4 Kohlenstoffatomen, einen Cyclohexylrest oder einen Phenylrest steht und

n die bereits genannte Bedeutung hat.

Typische Beispiele für geeignete Carbaminsäureester sind

1-(n-Butoxicarbonylamino)-3.3.5-trimethyl-5-(n-butoxicarbonylamino-methyl)-cyclohexan
1-Methyl-2.4-bis-(ethoxicarbonylamino)-benzol
1-Methyl-2.6-bis-(ethoxicarbonylamino)-benzol
1.10-Bis-(methoxicarbonylamino)-decan
1.12-Bis-(n-butoxicarbonylamino)-dodecan
1.12-Bis-(methoxicarbonylamino)-dodecan
1.12-Bis-(phenoxicarbonylamino)-dedecan
1.18-Bis-(n-butoxicarbonylamino)-octadecan
1.18-Bis-(benzyloxicarbonylamino)-octadecan
1.3-Bis-(ethoxicarbonylamino-methyl)-benzol
1.3-Bis-(methoxicarbonylamino)-benzol
1.3-Bis[(methoxicarbonylamino)-methyl]-benzol
1.3.6-Tris-(methoxicarbonylamino)-hexan
1.3.6-Tris-(phenoxicarbonylamino)-hexan
1.4′-Bis-[(2,4-dimethylphenoxi)carbonylamino]-butan
1.4-Bis-(ethoxicarbonylamino)-butan
1.4-Bis-(ethoxicarbonylamino)-cyclohexan
1.5-Bis-(ethoxicarbonylamino)-naphthalin
1.6-Bis-(ethoxicarbonylamino)-hexan
1.6-Bis-(methoxicarbonylamino)-hexan
1.6-Bis-(methoximethylcarbonylamino)-hexan
1.8-Bis-(ethoxicarbonylamino)-octan
1.8-Bis-(phenoxicarbonylamino)-4-(phenoxicarbonylaminmethyl)-octan
1.8-Bis-(propoxicarbonylamino)-octan
2.2-Bis-(4-propoxicarbonylamino-phenyl)-propan
2.2′-Bis-(methoxicarbonylamino)-diethylether

2.4′-Bis-(ethoxicarbonylamino)-diphenylmethan
2.4-Bis-(methoxicarbonylamino)-cyclohexan
4.4′-Bis-(ethoxicarbonylamino)-dicylcohexylmethan
4.4′-Bis-(ethoxicarbonylamino)-diphenylmethan
2.2-Bis[(4-methoxycarbonylamino)cyclohexyl]-propan
4.4′-Bis-(methoxicarbonylamino)-biphenyl
2.2-Bis-[(4-n-butoxycarbonylamino)cyclohexyl]-propan
4.4′-Bis-(phenoxicarbonylamino)-dicyclohexylmethan
4.4′-Bis-(phenoxicarbonylamino)-diphenylmethan

Für das erfindungsgemäße Verfahren geeignet sind auch Gemische der schon beispielhaft genannten 2.4′- bzw. 4.4′-Bis-(alkoxicarbonylamino)-diphenylmethane mit entsprechenden höherkernigen Homologen, in denen mehr als zwei Alkoxicarbonylamino-substituierte Benzolringe über Methylenbrücken miteinander verbunden sind. Solche "Carbamatgemische der Diphenylmethanreihe" entstehen beispielsweise bei der säurekatalysierten Kondensation von einfach alkoxicarbonylamino-substituierten Benzolen mit Formaldehyd.

Als Lösungsmittel, d.h. als Reaktionsmedium für die Durchführung des erfindungsgemäßen Verfahrens kommen polare Lösungsmittel in Betracht, die unter Spaltungs bedingungen einen oberhalb 150°C, vorzugsweise oberhalb 200°C liegenden Siedepunkt aufweisen oder überhaupt nicht unzersetzt destillierbar sind, und die im übrigen die folgenden Bedingungen erfüllen:

- Sie müssen unter den an späterer Stelle beschriebenen Extraktionsbedingungen sowohl die als Ausgangsmaterialien eingesetzten Carbaminsäureester als auch die als Reaktionsprodukte anfallenden Isocyanate lösen.
- Sie müssen unter den Spaltbedingungen thermisch weitgehend stabil sein.
- Sie müssen gegenüber den eingesetzten Carbaminsäureestern und den entstehenden Isocyanaten chemisch inert sein.
- Sie müssen mit den gemäß Stufe B) des erfindungsgemäßen Verfahrens eingesetzten Extraktionsmitteln mindestens eine Mischungslücke aufweisen.

Diesen Kriterien entsprechende, für das erfindungsgemäße Verfahren als Reaktionsmedium geeignete Lösungsmittel sind beispielsweise aliphatische Sulfone wie Diethylsulfon, Dipropylsulfon, Dibutylsulfon, Ethyl-propylsulfon; cyclische Sulfone wie Sulfolan, 2-Methylsulfolan, 3-Methylsulfolan, 2,4-Dimethylsulfolan; araliphatische Sulfone wie Methylphenylsulfon, Ethylphenylsulfon; aromatische Sulfone wie Diphenylsulfon, (4-Methylphenyl)-phenylsulfon; aromatische Nitroverbindungen wie Nitrobenzol, 2-Nitrotoluol, 3-Nitrotoluol, 4-Chlornitrobenzol; sowie Gemische derartiger Verbindungen. Bevorzugt werden Sulfolan, 3-Methylsulfolan und Nitrobenzol, besonders bevorzugt Sulfolan als Spaltungsmedium eingesetzt.

Als Extraktionsmittel eignen sich insbesondere aliphatische oder cycloaliphatische Kohlenwasserstoffe oder aliphatische Ether, die bei 1013 mbar einen Siedepunkt zwischen 30 und 200°C, vorzugsweise 30 bis 150°C aufweisen. Geeignet sind beispielsweise n-Hexan, i-Octan, den genannten Definitionen entsprechende Benzinfraktionen, Cyclohexan oder Methylcyclohexan, bzw. aliphatische Ether mit mindestens 4 Kohlenstoffatomen, vorzugsweise 4 bis 12 Kohlenstoffatomen, beispielsweise Diethylether, isomere Butylether, tert.-Butylmethylether oder n-Heptyl-methyl-ether. Aromatische Kohlenwasserstoffe wie Benzol, Toluol oder Xylol sind zwar ebenfalls geeignet, jedoch weniger bevorzugt. Die beispielhaft genannten aliphatischen oder cycloaliphatischen Kohlenwasserstoffe sind die besonders bevorzugten Extraktionsmittel. Beliebige Gemische der beispielhaft genannten Extraktionsmittel können selbstverständlich ebenfalls verwendet werden.

Die Durchführung des erfindungsgemäßen Verfahrens kann nach verschiedenen Verfahrensvarianten erfolgen. Im allgemeinen wird der zu spaltende Carbaminsäureester gegebenenfalls unter Zusatz von bis zu 10 Mol-%, vorzugsweise bis 1 Mol-% eines Katalysators als 1 bis 99 gew.-%ige, vorzugsweise 5 bis 90 gew.-%ige und besonders bevorzugt als 15 bis 75 gew.-%ige Lösung in einem, als Reaktionsmedium dienenden Lösungsmittel oder Lösungsmittelgemisch der oben genannten Art in einem geeigneten Reaktionsgefäß bei einem Druck von 0,001 bis 5 bar auf Temperaturen von 150 bis 350°C, vorzugsweise 150 bis 280°C erhitzt und die bei der Spaltung entstehenden Alkoholdämpfe gegebenenfalls über einen Dephlegmator über Kopf abdestilliert. Um den bei der Spaltung entstehenden Alkohol möglichst rasch und effektiv aus dem Spaltreaktor zu entfernen, kann es zweckmäßig sein, durch das Reaktionsgemisch ein inertes Gas oder eine unter Normalbedingungen niedrigsiedende inerte Flüssigkeit, deren Trennung vom Alkohol keine Probleme bereitet, zu leiten.

Die Spaltungsreaktion kann in an sich bekannten Apparaturen kontinuierlich, batchweise oder diskontinuierlich durchgeführt werden.

Wenn als Reaktionsmischung eine bis zu 30 gew.-%ige Lösung des Carbaminsäureesters in einem Lösungsmittel der oben genannten Art eingesetzt wird, erfolgt die Spaltungsreaktion bevorzugt kontinuierlich in kaskadenartig angeordneten Kesseln, die so gewählt werden, daß eine ausreichende Verweilzeit der eingespeisten Lösung zur Erreichung einer weitgehenden Spaltung des Carbaminsäureesters gewährleistet ist. In Abhängigkeit von der Reaktivität des zu spaltenden Carbaminsäureesters und der angewandten Reaktionstemperatur kann die Reaktionszeit von wenigen Minuten bis mehrere Stunden betragen.

Bevorzugt werden die Bedingungen so gewählt, daß bei Reaktionszeiten zwischen 30 Minuten und 5 Stunden Umsetzungsgrade von mindestens 10 % der Theorie, vorzugsweise mehr als 50 % der Therorie erreicht werden. Aufgrund der niedrigen Konzentration in der Reaktionsmischung wird die Bildung von polymeren Nebenprodukten weitgehend vermieden.

Bei Einsatz von Lösungen, deren Carbaminsäurekonzentration über 30 Gew.-% liegt, hat es sich als vorteilhaft erwiesen, die Spaltungsreaktion durchzuführen, indem man die Lösung in einer dünnen Schicht an der Innenwand eines beheizten Rohres entlangführt. Die Verweilzeit der Lösung im Reaktionsrohr wird dabei zur Unterdrückung von Nebenreaktionen sehr kurz gehalten. Der bei der Spaltungsreaktion frei werdende Alkohol wird als gasförmiges Produkt über Kopf abgenommen, die isocyanathaltige Reaktionsmischung wird als Sumpfprodukt ausgeschleust. Die Verteilung der eingespeisten Reaktionsmischung auf der Rohrinnenwand kann in senkrecht stehenden Rohrreaktoren ohne Hinzuziehung besonderer Vorrichtungen geschehen, sofern die Reaktionsmischung über eine geeignete Vorrichtung, z.B. eine Düse, gleichmäßig auf die Rohrwandung aufgetragen wird. Sie kann aber auch unter Zuhilfenahme eines mechanischen Rührwerks oder ähnlicher Einrichtungen bewerkstelligt werden. In nicht senkrecht stehenden Rohrreaktoren ist die Verwendung eines Rührwerks oder einer anderen geeigneten Vorrichtung in den meisten Fällen notwendig.

Zur Beschleunigung der Spaltungsreaktionen können, wie bereits angedeutet, der Reaktionsmischung die an sich bekannten Spaltungskatalysatoren, wie sie beispielsweise in DE-OS 26 35 490 oder US-PS 39 19 279 beschrieben sind, zugesetzt werden.

Die erste Stufe des erfindungsgemäßen Verfahrens kann bei Unter- und Überdruck im Bereich von 0,001 bis 5 bar durchgeführt werden. Um eine rasche Entfernung des Alkohols aus dem Reaktionsgemisch zu gewährleisten, wird die Spaltung bevorzugt bei Unterdrücken im Bereich von 0,005 bis 0,5 bar durchgeführt.

Die Spaltungsreaktion erfolgt unter solchen Temperatur-und Druckbedingungen innerhalb der obengenannten Bereiche, bei denen der abgespaltene Alkohol die einzige, das Reaktionsgemisch gasförmig verlassende Komponente darstellt. Dies kann nicht nur durch geeignete Wahl der Spaltungstemperatur, sondern insbesondere auch durch geeignete Wahl der Temperatur des Dephlegmators sichergestellt werden.

Für die Durchführung des erfindungsgemäßen Verfahrens ist es wichtig, daß sowohl die zur Spaltung eingesetzten Carbaminsäureester als auch die bei der Reaktion entstehenden Isocyanate unter den Bedingungen der der Spaltung folgenden Extraktion in gelöster Form vorliegen.

Die bei der thermischen Spaltung von Carbaminsäureestern entstehenden Rohlösungen, die vorwiegend die herzustellenden Polyisocyanate, daneben aber auch Reste von nicht oder nur teilweise gespaltenen Carbaminsäureestern enthalten, werden auf eine unterhalb der Spaltungstemperatur liegende Temperatur abgekühlt und in einem zweiten Verfahrensschritt einer Extraktion unterworfen. Falls erwünscht, kann die Rohlösung selbstverständlich zuvor durch fraktionierte Destillation aufkonzentriert werden.

Zur Durchführung des erfindungsgemäßen Extraktionsschritts wird die isocyanathaltige Rohlösung mit einem bei Raumtemperatur flüssigen Extraktionsmittel der oben beispielhaft genannten Art intensiv durchmischt, wobei, bezogen auf die zu extrahierende Rohlösung, das Extraktionsmittel in einer 0,1 bis 25-fachen, vorzugsweise 0,5- bis 5-fachen Gewichtsmenge zum Einsatz gelangt.

Die Durchmischung der Rohlösung mit dem Extraktionsmittel erfolgt im allgemeinen innerhalb des Temperaturbereiches von -20°C bis 150°C, vorzugsweise bei 10°C bis 100°C.

Im allgemeinen entsteht hierbei spontan ein zweiphasiges, aus zwei flüssigen Phasen bestehendes Gemisch, welches nach Phasenabscheidung in eine spezifisch leichtere und eine spezifisch schwerere Phase getrennt werden kann. Die Bildung eines zweiphasigen Systems kann in besonderen Fällen jedoch auch durch eine Abkühlung des Gemischs der Rohlösung mit dem Extraktionsmittel begünstigt werden. So ist es beispielsweise möglich, die Durchmischung bei ca. 70°C bis 100°C vorzunehmen und an schließend das Gemisch auf eine tiefere Temperatur beispielsweise im Temperaturbereich von 10°C bis 40°C abzukühlen. In dem sich bildenden zweiphasigen System bildet die spezifisch leichtere Phase im allgemeinen die Hauptphase und die spezifisch schwerere Phase im allgemeinen die Nebenphase, wobei das Volumenverhältnis weitgehend von der Menge des eingesetzten Extraktionsmittels abhängt. Die Phasentrennung kann bei der Durchführung des erfindungsgemäßen Verfahrens nach an sich bekannten Methoden, beispielsweise durch Ablassen der schwereren Phase, Dekantieren, Abhebern oder andere geeignete Methoden der Phasentrennung vorgenommen werden. Ein Teil des in reiner Form zu gewinnenden Polyisocyanats liegt dann in der spezifisch leichteren Hauptphase vor. Weitere Bestandteile der spezifisch leichteren Phase sind ein Teil des als Spaltmediums verwendeten Lösungsmittels sowie die Hauptmenge des eingesetzten Extraktionsmittels. Die spezifisch schwerere Phase besteht hauptsächlich aus dem als Spaltmedium verwendeten Lösungsmittel, den nicht oder nur teilweise umgesetzten Carbaminsäureestern sowie den sich während der Spaltungsreaktion gebildeten Nebenprodukten und dem nicht in die spezifisch leichtere Phase übergegangenen Teil des zu gewinnenden Polyisocyanats. Um auch diesen Teil an Polyisocyanat in reiner Form zu gewinnen, kann die spezifisch schwerere Phase einer oder mehrerer erneuter Extraktionen in der beschriebenen Art und Weise unterworfen werden.

Gemäß einer besonderen Ausführungsform des erfindungsgemäßen Verfahrens erfolgt die Durchmischung der Rohlösung mit dem Extraktionsmittel und die anschließende Phasentrennung, d.h. die Extraktion der Rohlösung kontinuierlich unter Verwendung der üblichen, kontinuierlich arbeitenden Gegenstromextraktionsapparaturen.

Als Ergebnis der Extraktion resultieren eine oder mehrere, gegebenenfalls zu vereinigende, spezifisch leichtere Extraktionsphasen, die das zu gewinnende Polyisocyanat enthalten, und eine, in der Regel homogene, zweite Phase, die vorwiegend nicht oder nur unvollständig umgesetzte Carbaminsäureester enthält.

Die spezifisch schwerere Phase kann in der Spaltungsreaktion als Lösungsmittel wiederverwendet werden, wobei es sich als zweckmäßig erwiesen hat, einen Teil der Phase auszuschleusen und gegen neues Lösungsmittel zu ersetzen, um eine Anreicherung der in der schwereren Phase befindlichen Nebenprodukte zu vermeiden.

Zur Gewinnung der Polyisocyanate in reiner Form werden die genannten spezifisch leichteren Extraktphasen destillativ aufgearbeitet, wobei im allgemeinen das Extraktionsmittel die zunächst destillativ zu entfernende Fraktion darstellt. Auch die Trennung der Polyisocyanate von Restmengen an Spaltungslösungsmitteln kann destillativ erfolgen, wobei das Polyisocyanat oder das als Spaltungsmedium verwendete Lösungsmittel den Destillationsrückstand bildet. Vorzugsweise wird man solche Spaltungslösungsmittel der oben beispielhaft genannten Art verwenden, die sich bezüglich ihres Siedeverhaltens ausreichend deutlich vom zu gewinnenden Polyisocyanat unterscheiden, so daß eine einwandfreie Auftrennung möglich ist. Im allgemeinen stellt das zu gewinnende Polyisocyanat bei der destillativen Aufarbeitung der spezifisch leichteren Extraktphasen den Destillationsrückstand dar. Auch die destillative Aufarbeitung kann kontinuierlich unter Verwendung der bekannten Destillationsapparaturen erfolgen. Die im allgemeinen als Destillationsrückstand anfallenden Polyisocyanate können gegebenenfalls einer weiteren Feindestillation unterzogen werden. Jedoch auch ohne derartige Feindestillationen weisen die als Destillationsrückstände anfallenden Polyisocyanate bereits einen Reinheitsgrad von zum Teil über 90 Gew.-% auf.

Es ist ein besonderer Vorteil des erfindungsgemäßen Verfahrens, daß die bei der Spaltungsreaktion entstehenden Polyisocyanate nicht durch eine Destillation, die mit einer unnötigen thermischen Belastung verbunden ist, sondern unter schonenden Bedingungen durch Extraktion von gegebenenfalls entstandenen Nebenprodukten abgetrennt werden. Dies hat zur Folge, daß die an sich bekannten Folgereaktionen von Isocyanaten, die unter Umständen von den bei der Spaltungsreaktion gebildeten Nebenprodukten und den eingesetzten Carbaminsäureestern katalysiert werden, weitgehend unterdrückt werden und somit ein wesentlich größerer Teil des bei der Spaltungsreaktion gebildeten Polyisocyanats unzersetzt erhalten bleibt und in reiner Form isoliert werden kann.

Die nachfolgenden Beispiele dienen der weiteren Erläuterung des erfindungsgemäßen Verfahrens. Alle Prozentangaben beziehen sich, falls nichts anderslautendes vermerkt, auf Molprozente.

Die Ausbeute nach einem ersten Zyklus bedeutet die tatsächliche Ausbeute, die in den Beispielen erhalten wird. Die Ausbeute für kontinuierliche Fahrweise bezieht sich auf die Gesamtmenge an Diisocyanat, die dadurch erhalten wird, daß (1) zusätzliches Diisocyanat aus dem ersten Zyklus gewonnen wird und (2) unreagierte und teilweise reagierte Carbaminsäureester in nachfolgenden Zersetzungsschritten in Produkt umgewandelt werden. Diese Ausbeute berechnet sich als Grenzwert einer geometrischen Reihe wie folgt:

$$ Y = \frac{Y_1}{1 - (S_1 - Y_1)/100} $$

mit Y = Ausbeute für kontinuierliche Fahrweise (%)
$Y_1$ = Ausbeute nach einem ersten Zyklus (%)
$S_1$ = Summe der Gesamtmenge an Diisocyanat sowie nicht reagiertem und teilweise reagiertem Carbaminsäureester (%)

Variante A):

In einem Kolben, der mit einem mit Glas umhüllten Blattrührer, einem Thermometer, einem unter die Flüssigkeits oberfläche geführten Stickstoffeinlaß und einem Dephlegmator ausgerüstet ist, wird das Lösungsmittel auf etwa 20° C unterhalb der gewünschten Spaltungstemperatur erhitzt, der zu spaltende Carbaminsäureester sowie gegebenenfalls Katalysatoren und/oder Stabilisierungsmittel zugegeben und der gesamte Kolbeninhalt unter kräftigem Rühren und Durchleiten eines konstanten Stickstoffstroms auf die gewünschte Spaltungstemperatur erhitzt. Das Vakuum wird so eingestellt, daß sich im Dephlegmator kräftiger Rückfluß einstellt. Die Temperatur des Dephlegmators soll zwischen dem Siedepunkt des verwendeten Lösungsmittels und dem des zu entfernenden Alkohole liegen. Am oberen Ende des Dephlegmators werden die anfallenden Spaltgase mittels eines wassergekühlten Liebigkühlers (teilweise) kondensiert und in einer gegebenenfalls mit Kältemischung (Aceton/Trockeneis) gekühlten Vorlage aufgefangen.

Der Umsetzungsgrad der Spaltungsreaktion wird dadurch bestimmt, daß mittels einer durch ein Septum eingeführten Spritze kleine Proben der Reaktionslösung entnommen und titrimetrisch (Umsetzung mit Dibutylamin und Rücktitration des überschüssigen Amine mit Salzsäure) deren Isocyanatgehalt bestimmt wird.

Die Spaltungereaktion wird nach Erreichen der gewünschten Spaltrate abgebrochen, indem die Reaktionslösung auf eine für die Extraktion geeignete Temperatur abgekühlt wird, und in der weiter unten beschriebenen Art und Weise einer Extraktion unterworfen.

Variante B):

Als Spaltungsreaktor dient ein zylinderförmiger Dünnschichtverdampfer (effektive Länge 300 mm; Durchmesser 35 mm), der mit einem aus Metall gearbeiteten Flügelblattrührer ausgestattet ist, wobei dessen bewegliche Flügel bis an die Wandung des Dünnschichtverdampfers reichen. Zur Einspeisung des zu spaltenden Carbaminsäureesters dient ein am Kopf des Dünnschichtverdampfers angebrachter beheizbarer Tropftrichter. Die Ausschleusung nicht verdampfbarer Reaktionsprodukte erfolgt über einen am Fuß des Dünnschichtverdampfers angebrachten Absperrhahn, die Entnahme der verdampfbaren Komponenten des Reaktionsgemischs erfolgt am Kopf des Dünnschichtverdampfers über einen beheizten Querstromkühler, auf dessen oberen Ende ein Schlangenkühler mit Entnahmeboden aufgesetzt ist. Die Evakuierung der Spaltungsapparatur erfolgt über eine hinter dem Schlangenkühler angebrachte Kühlfalle mittels einer Drehschieberpumpe.

Die während der Spaltungsreaktion als Sumpf anfallende isocyanathaltige Mischung wird, gegebenenfalls nach Zugabe von weiterem Lösungsmittel, auf eine für die Extraktion geeignete Temperatur gebracht und nach der im folgenden beschriebenen Methode extrahiert.

Extraktion:

In einem beheizbaren Kolben mit Bodenablaß, der mit einem mit Glas umhüllten Blattrührer, einem Thermometer und einem Rückflußkühler ausgerüstet ist, wird die zu extrahierende Mischung extrahiert. Dazu wird sie mit dem Extraktionsmittel vermischt und 30 Minuten innig gerührt. Nach 10 minütigem Stehenlassen werden die beiden gebildeten Phasen getrennt. Die spezifisoh schwerere Phase wird in manchen Beispielen einer oder mehreren weiteren Extraktionen mit frischem Extraktionsmittel unterworfen. Die spezifisch leichteren Phasen werden vereint und, ebenso wie die nach der Extraktion übrigbleibende schwerere Phase, mittels HPLC auf ihre Zusammensetzung geprüft.

In den nachstehend benutzten Abkürzungen bedeuten "DI" urethanfreie Polyisocyanate, insbesondere Diisocyanat, "IU" teilgespaltenes, Urethan- und Isocyanatgruppen aufweisendes Produkt, insbesondere Isocyanatourethan, und "DU" unverändertes Ausgangsmaterial, insbesondere Diurethan.

Beispiel 1

Gemäß Variante A) wird eine Lösung von 151 g 1,5-Bis-(ethoxycarbonylamino)-naphthalin (NDU) und 2,60 g Dibutylzinndilaurat in 1346 g Sulfolan bei 200 °C 300 Minuten lang thermolysiert. Das Reaktionsgemisch, dessen NCO-Wert bei 2,26 Gew.-% (80,7 % d. Th.) liegt, wird auf 70 °C abgekühlt und viermal mit je 4700 g Cyclohexan extrahiert. Die gesammelten Extrakte enthalten laut HPLC-Analyse 58,9 g 1,5-Diisocyanatonaphthalin (NDI) sowie 23,9 g 1-Ethoxycarbonylamino-5-isocyanatonaphthalin (NIU). Der NDU-Gehalt liegt unterhalb der Nachweisgrenze. In der Sulfolanphase befinden sich nach der Extraktion 15,6 g NIU sowie 2,4 g NDU. Der NDI-Gehalt liegt unterhalb der Nachweisgrenze. Daraus errechnet sich hinsichtlich der Bildung und Isolierung von 1,5-Diisocyanatonaphthalin (NDI) eine Ausbeute von 56,0 % nach einem ersten Zyklus und eine Ausbeute von 80,6 % bei kontinuierlicher Fahrweise.

Beispiel 2

Gemäß Variante B) wird innerhalb von 6,5 Stunden über den auf 100 °C thermostatisierten Tropftrichter eine Lösung von 225 g 2,4-Bis-(ethoxycarbonylamino)-toluol (TDU), 0,55 g Dibutylzinndilaurat und 2,2 g Stearinsäurechlorid in 75 g Sulfolan und 30 g Chlorbenzol (Tropfgeschwindigkeit: 50 g/h) in den auf 290 °C erhitzten Dünnschichtverdampfer eingespeist. Der Druck in der Apparatur beträgt während der Spaltung 200 mbar. Es werden 261 g Sumpfprodukt erhalten, in dem laut HPLC 70,7 g TDU, 99,2 g TIU (Isomerengemisch) sowie 24,7 g TDI enthalten sind. Der Sumpf wird bei 50 °C viermal mit je 260 g Isooctan extrahiert. Nach der letzten Extraktion werden in der Sulfolanphase 65,7 g TDU, 85,7 g TIU sowie 4,7 g TDI nachgewiesen. Die gesammelten Extrakte enthalten insgesamt 2,0 g TDU, 15,8 g TIU sowie 19,4 g TDI. Daraus errechnet sich hinsiohtlich Bildung und Isolierung von 2,4-Diisocyanatotoluol (TDI) eine Ausbeute von 13,3 % nach einem ersten Zyklus und eine Ausbeute von über 98 % für kontinuierliche Fahrweise.

Beispiel 3

Gemäß Variante A) wird eine Lösung von 85,6 g 4,4'-Bis-(ethoxycarbonylamino)-diphenylmethan-Homologen-Gemisch (Polymer-MDU), dessen MDU-Gehalt insgesamt bei 73,5 Gew.-% liegt, und 0,26 g Dibutylzinndilaurat in 1414 g Sulfolan bei 250 °C 70 Minuten lang thermolysiert. Das Reaktionsgemisch, dessen NCO-Wert bei 0,97 Gew.-% (94,4 % d. Th.) liegt, wird auf 20 °C abgekühlt, viermal mit je 1740 g tert.-Butylmethylether/Isooctan-Gemisch (1:1) extrahiert. Die gesammelten Extrakte enthalten laut MPLC-Analyse 28,7 g Polymer-MDI, 1,0 g Polymer-MIU sowie 0,9 g Polymer-MDU. In der Sulfolanphase befinden sich nach der Extraktion 12,5 g Polymer-MDI, 3,0 g Polymer-MIU sowie 0,7 g Polymer-MDU. Daraus errechnet sich hinsichtlich der Bildung und Isolierung von 4,4'-Diisocyanatodiphenylmethan-Homologen-Gemisch (Polymer-MDI) eine Ausbeute von 61,9 % nach einem ersten Zyklus und eine Ausbeute von 98,3 % bei kontinuierlicher Fahrweise.

Beispiel 4

Gemäß Variante B) wird innerhalb von 6,25 Stunden über den auf 125 °C thermostatisierten Tropftrichter eine Lösung von 140 g 4,4'-Bis-(ethoxycarbonylamino)-diphenylmethan (MDU) und 0,35 g Dibutylzinndilaurat in 140 g Sulfolan und 28 g Chlorbenzol (Tropfgeschwindigkeit: 50 g/h) in den auf 260 °C erhitzten Dünnschichtverdampfer eingespeist. Der Druck in der Apparatur beträgt während der Spaltung 150 mbar. Es werden 261 g Sumpfprodukt erhalten, in dem laut HPLC 51,7 g MDU, 38,6 g MIU sowie 28,4 g MDI enthalten sind. Der Sumpf wird bei 80 °C viermal mit je 260 g Cyclooctan extrahiert. Nach der letzten Extraktion werden in der Sulfolanphase 49,3 g MDU, 26,8 g MIU sowie 5,5 g MDI nachgewiesen. Die gesammelten Extrakte enthalten insgesamt 2,8 g MDU, 10,8 g MIU sowie 25,4 g MDI. Daraus errechnet sich hinsichtlich Bildung und Isolierung von 4,4′-Diisocyanatodiphenylmethan (MDI) eine Ausbeute von 24,8 % nach einem ersten Zyklus und eine Ausbeute von 94,7 % für kontinuierliche Fahrweise.

### Beispiel 5

Gemäß Variante A) wird eine Lösung von 75 g 1-(Ethoxycarbonylamino)-3,3,5-trimethyl-5-(ethoxycarbonylaminomethyl)-cyclohexan (IPDU) und 0,26 g Dibutylzinndilaurat in 1425 g Sulfolan bei 250° C 60 Minuten lang thermolysiert. Das Reaktionsgemisch, dessen NCO-Wert bei 1,13 Gew.-% (84,9 % d. Th.) liegt, wird auf 20° C abgekühlt und viermal mit je 860 g Cyclohexan extrahiert. Die gesammelten Extrakte enthalten laut HPLC-Analyse 34,8 g IPDI sowie 2,0 g IPIU (Isomerengemisch). (Der bei der Spaltungsreaktion unverändert gebliebene Ausgangscarbaminsäureester (IPDU) wird in der HPLC-Analyse nicht erfaßt). In der Sulfolanphase befinden sich nach der Extraktion 7,0 g IPDI sowie 5,5 g IPIU. Daraus errechnet sich hinsichtlich der Bildung und Isolierung von 1-Isocyanato-3,3,5-trimethyl-5-(isocyanatomethyl)cyclohexan (IPDI) eine Ausbeute von 66,0 % nach einem ersten Zyklus und eine Ausbeute von über 88 % bei kontinuierlicher Fahrweise.

### Beispiel 6

Gemäß Variante B) wird innerhalb von 9 Stunden über den auf 100° C thermostatisierten Tropftrichter eine Lösung von 112 g 2,4-Bis-(ethoxycarbonylamino)-toluol (TDU), 0,27 g Dibutylzinndilaurat und 1,1 g Stearinsäurechlorid in 37 g Sulfolan und 15 g Chlorbenzol (Tropfgeschwindigkeit: 18 g/h) in den auf 290° C erhitzten Dünnschichtver dampfer eingespeist. Der Druck in der Apparatur beträgt während der Spaltung 200 mbar. Es werden 123 g Sumpfprodukt erhalten, in dem laut HPLC 16,0 g TDU, 34,5 g TIU (Isomerengemisch) sowie 30,7 g TDI enthalten sind. Der Sumpf wird bei 20° C viermal mit je 125 g Cyclooctan extrahiert. Nach der letzten Extraktion werden in der Sulfolanphase 15,1 g TDU, 28,4 g TIU sowie 5,9 g TDI nachgewiesen. Die gesammelten Extrakte enthalten insgesamt 0,6 g TDU, 5,5 g TIU sowie 25,0 g TDI. Daraus errechnet sich hinsichtlich Bildung und Isolierung von 2,4-Diisocyanatotoluol (TDI) eine Ausbeute von 34,2 % nach einem ersten Zyklus und eine Ausbeute von 82,8 % für kontinuierliche Fahrweise.

**Ansprüche**

1. Verfahren zur Herstellung von Polyisocyanaten durch thermische Spaltung der den Polyisocyanaten entsprechenden N-substituierten Carbaminsäureester in Lösung in einem als Spaltmedium dienenden Lösungsmittel oder Lösungsmittelgemisch bei oberhalb 150° C liegenden Temperaturen und unter fortlaufender destillativer Entfernung des bei der Spaltung entstehenden Alkohols, dadurch gekennzeichnet, daß man

A) den Carbaminsäureester in einem Lösungsmittel oder Lösungsmittelgemisch löst, das

a) die Carbaminsäureester zu lösen vermag,

b) bei der Spaltungstemperatur stabil und gegenüber den Carbaminsäureestern und den gebildeten Isocyanaten inert ist, und

c) mit dem gemäß B) eingesetzten Extraktionsmittel mindestens eine Mischungslücke aufweist, und

B) das im Spaltmedium verbleibende, gegebenenfalls durch fraktionierte Destillation im Sumpf angereicherte Polyisocyanat durch Extraktion mit einem Extraktionsmittel, welches mit dem Spaltmedium nicht unbegrenzt mischbar ist und für das Polyisocyanat ein Lösungsmittel darstellt, aus dem Spaltmedium entfernt und gegebenenfalls anschließend die dabei anfallende Lösung des Polyisocyanats in dem Extraktionsmittel destillativ aufarbeitet, und

C) den nicht extrahierten Teil des Reaktionsgemisches zurückführt, vorzugsweise in die Spaltungsreaktion.

2. Verfahren gemäß Anspruch 1, dadurch gekennzeichnet, daß als Spaltmedium ein polares, unter den Spaltungsbedingungen einen Siedepunkt von oberhalb 150° C aufweisendes oder nicht unzersetzt destillierbares Lösungsmittel oder ein Gemisch derartiger Lösungsmittel eingesetzt wird.

3. Verfahren gemäß Anspruch 1 und 2, dadurch gekennzeichnet, daß als Spaltmedium Sulfolan oder 3-Methyl-sulfolan eingesetzt wird.

4. Verfahren gemäß Anspruch 1 bis 3, dadurch gekennzeichnet, daß als Extraktionsmittel Lösungsmittel mit einem bei 1013 mbar bei 30 bis 200° C liegenden Siedepunkt bzw. -bereich, ausgewählt aus der Gruppe der aliphatischen, cycloaliphatischen oder araliphatischen Kohlenwasserstoffe, aliphatischen Ether oder beliebigen Gemischen derartiger Lösungsmittel, eingesetzt werden.

5. Verfahren gemäß Anspruch 1 bis 4, dadurch gekennzeichnet, daß als Extraktionsmittel Isooctan, Cyclohexan, Toluol und/oder tert.-Butyl-methylether eingesetzt wird.